# EUROPEAN PATENT APPLICATION

(11) **EP 2 959 889 A1**
(43) Date of publication of application: **30.12.2015**
(21) Application number: 15173520.6
(22) Date of filing: 24.06.2015
(51) Int. Cl.: A61K 9/20, A61K 31/192, A61K 9/46

(54) **ORALLY DISINTEGRATING FORMULATIONS OF LOXOPROFEN**

(30) Priority: 25.06.2014 TR 201407411
(71) Applicant: Sanovel Ilac Sanayi ve Ticaret A.S., 34460 Istanbul (TR)
(72) Inventor: CIFTER, Ümit, 34460 Istanbul (TR); TÜRKYILMAZ, Ali, 34460 Istanbul (TR); ERDEM, Yelda, 34460 Istanbul (TR); UCAR, Ezgi, 34460 Istanbul (TR)
(74) Representative: Sevinç, Erkan

(57) **Abstract**

The present invention relates to a pharmaceutical formulation comprising a therapeutically effective amount of loxoprofen or a pharmaceutically acceptable salt thereof in the form of orally disintegrating tablet.

## Description

### Field of Invention

The present invention relates to a pharmaceutical formulation comprising a therapeutically effective amount of loxoprofen or a pharmaceutically acceptable salt thereof in the form of orally disintegrating tablet.

### Background of Invention

Loxoprofen is a non-steroidal anti-inflammatory drug in the propionic acid derivatives group. It is a prodrug and it is quickly converted to its active trans-alcohol metabolite following oral administration. It is a non-selective cyclooxygenase inhibitor and works by reducing the synthesis of prostaglandins from arachidonic acid. Its chemical name is (RS)-2-{4-[(2-oxocyclopentyl)methyl]phenyl}propanoic acid and its chemical structure is shown in the Formula I.

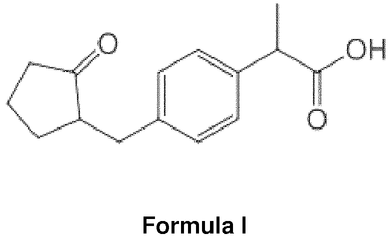

The patent application US4161538 (A) discloses the loxoprofen molecule.

The patent JP2669517 (B2) discloses a solid preparation containing loxoprofen sodium and additives such as microcrystalline cellulose, hydroxypropyl cellulose having low substitution degree, lactose or magnesium stearate, in the form of a tablet, capsule, granule or powder.

The patent application JP2010270140 (A) discloses an oral pharmaceutical formulation which alleviates gastric mucosa disorders caused by loxoprofen, comprises one or more sugars and sugar alcohols selected from the group consisting of sucrose, maltitol, fructose, xylitol, trehalose, lactose and lactitol; and loxoprofen. A tablet, subtle granule (powder is included), capsule, solution (syrup is included), etc., may be cited as a concrete dosage form of the oral pharmaceutical formulation.

In the state of art, tablet dosage form is commercially available under the brandname LOXONIN® in strength of 60 mg loxoprofen. It is recommended 3 times a day dose for adults. However, there is no orally disintegrating tablet formulation of loxoprofen available in the market.

Over the past decades, orally disintegrating tablets (ODTs) have gained considerable attention as a preferred alternative to conventional tablets and capsules due to better patient compliance. ODTs are solid dosage forms containing active ingredients which disintegrate rapidly through buccal mucosa. It is desirable in the treatment of a number of diseases. They are also advantageous for administrations of medicaments to patients who are traveling or have little access to water are similarly affected or patients who are mentally retarded, uncooperative, nauseated, or patients who have difficulties swallowing other dosage forms.

With all these advantages, it is also difficult to develop orally disintegrating formulations because of several different reasons and requirements such as rapid disintegration and stability. Dosage form must disintegrate in the oral cavity with the existence of saliva in a short period of time. So those formulations should have a porous structure. However, these porous characteristic tend to be very sensitive to humidity and may be lead to instability problems.

To fulfill all these requirements, the formulation for loxoprofen needs to be adapted in particular by a careful selection of the excipients which give a high porous structure with high stability and suitable disintegration time.

### Description of the invention

The present invention provides an orally disintegrating formulation comprising loxoprofen or a pharmaceutically acceptable salt thereof and one or more pharmaceutically acceptable excipients.

The main embodiment of this present invention is to obtain rapidly disintegrating and stable formulations that comprise loxoprofen.

An embodiment of this present invention provides an orally disintegrating formulation comprising loxoprofen or a pharmaceutically acceptable salt thereof and croscarmellose sodium as a super disintegrant is present in an amount of 5 to 30 %, more preferably it is 5 - 15 % by weight of total formulation and one or more pharmaceutically acceptable excipients,

According to one embodiment of this present invention is to provide an orally disintegrating formulation wherein loxoprofen or a pharmaceutically acceptable salt thereof is present in an amount of about 5 to 80 % by weight of total formulation, preferably it is about 20 to 60 % by weight of total formulation.

Another embodiment of the present invention is to provide a formulation comprising croscarmellose sodium as a super-disintegrant which provides porous structure and orally disintegration.

As used herein, the term "super-disintegrant" is defined as the pharmaceutical ingredient that provides improved compressibility, compatibility and substantially faster disintegration than the conventional disintegrants. According to this embodiment, the term "super-disintegrant" is croscarmellose sodium,

In one embodiment, the weigth ratio of loxoprofen to croscarmellose sodium is in the range of 0.1 to 50 (w/w), preferably 2 to 30 (w/w) more preferably it is 2 to 20 (w/w). It has been surprisingly found that with this specific ratio, orally disintegrating formulation having a desired disintegration time has been achieved. According to this embodiment, the formulation of loxoprofen is disintegrated in less than 1 min.

In this invention, croscarmellose has been chosen as a super-disintegrant. Due to its chemical and physical properties, it forms a porous structure which provides rapid oral disintegration of the tablet. The amount of croscarmellose is present in an amount of 0.1 to 50 %, preferably 5 to 30 %, more preferably it is 5 - 15 % by weight of total formulation.

The orally disintegrating formulation of this present invention comprising at least one pharmaceutically acceptable excipient selected from the group comprising acidifying agents, alkalizing agents, diluents, taste masking coating, binders, lubricants, glidants, sweeteners and flavouring agents.

In one embodiment, to further improve the disintegration and taste of the formulation and acidifying agents and alkalizing agents are used. Acidifying agents and alkalizing agents reacts rapidly in the presence of water by releasing carbon dioxide. Choice of acidifying agent - alkalizing agent couple and their ratio is important in terms of taste, disintegration time and water solubility of the formulation. In this invention, the ratio of the acidifying agents to the alkalizing agents is between 20:1 (w/w) to 1:20 (w/w), preferably 10:1 (w/w) to 1:10 (w/w) and more preferably it is 5:1 (w/w) and 1:5 (w/w).

The acidifying agent is selected from the group comprising citric acid, fumaric acid, adipic acid, acetic acid, hydrochloric acid, malic acid, nitric acid, phosphoric acid, sulfuric acid, tartaric acid and mixtures thereof, preferably it is citric acid.

The alkalizing agent is selected from the group comprising sodium bicarbonate, sodium glycine carbonate, ammonia solution, ammonium carbonate, diethanolamine, diisopropanolamine, potassium hydroxide, sodium carbonate, sodium borate, sodium hydroxide, trolamine and mixtures thereof, preferably it is sodium bicarbonate.

In this embodiment, the amount of citric acid is in the range of 2 - 90 %, preferably 5 - 60 %, more preferably it is 10 - 40 % by weight of total formulation.

In this embodiment, the amount of sodium bicarbonate is in the range of 2 - 90 %, preferably 5 - 60 %, more preferably it is 10 - 40 % by weight of total formulation.

The diluents are selected from the group comprising mannitol, spray-dried mannitol, microcrystalline cellulose, lactose, starch, sodium carbonate, sodium bicarbonate, calcium carbonate and mixtures thereof; preferably the diluents is mannitol.

Porous structure achieved with croscarmellose sodium has caused instability in the formulation. Moreover, acidifying agents and alkalizing agents reacts rapidly in the presence of water and it leads moisture sensitivity during the shelf life. In further, to prevent moisture sensitivity of this present formulation, mannitol has been used. The amount of mannitol is in the range of 1.0 to 60.0% by weight of total formulation; preferably it is 10.0 to 40.0% by weight of total formulation. It is surprisingly found that with this ratio stable orally disintegrating tablet of loxoprofen has been achieved.

In one embodiment, to further improve the taste of loxoprofen taste-masking coating is used. Taste-masking coating is selected from the group comprising aminoalkyl metacrylate, carboxymethylcellulose calcium, carboxymethylcellulose sodium, carnauba wax, cellulose acetate, cellulose acetate phthalate, ceresin, cetyl alcohol, chitosan, ethylcellulose, fructose, gelatin, glycerin, glyceryl behenate, glyceryl palmitostearate, hydroxyethyl cellulose, hydroxyethylmethyl cellulose, hydroxypropyl cellulose, hypromellose, hypromellose phthalate, isomalt, liquid glucose, maltitol, maltodextrin, methylcellulose, microcrystalline wax, paraffin, poloxamer, polydextrose, polyethylene glycol, polyethylene oxide, poly-DL-(lactic acid), polyvinyl acetate phthalate, polyvinyl alcohol, potassium chloride, povidone, shellac, shellac with stearic acid, sucrose, surface color agents, titanium oxide, tributyl citrate, triethyl citrate, vanillin, white wax, xylitol, yellow wax, zein, dimethylaminoethyl methacrylate, butyl methacrylate and methyl methacrylate (Eudragit E 100) (Poly(butyl methacrylate-co-(2-demethylaminoeethyl)methacrylate-co-methyl methacrylate)) or mixture of polyethylene glycol and polyvinyl alcohol (Kollicoat IR) and their copolymers and their mixtures.

The binders are selected from the group comprising povidone, carbomers, carboxymethylcellulose sodium, cellulose acetate phthalate, chitosan, copovidone, corn starch and pregelatinized starch, dextrates, dextrin, dextrose, ethylcellulose, gelatin, glyceryl behenate, guar gum, hydrogenated vegetable oil type I, hydroxyethyl cellulose, hydroxyethylmethyl cellulose, hydroxypropyl cellulose, hydroxypropyl starch, hypromellose, liquid glucose, magnesium aluminum silicate, maltodextrin, maltose, methylcellulose, pectin, poloxamer, polycarbophil, polydextrose, polyethylene oxide, polymethacrylates, sodium alginate, starch, pregelatinized starch, stearic acid, sucrose or mixtures thereof.

The lubricants are selected from the group comprising sodium stearyl fumarate, magnesium strearate, calcium stearate, sodium lauryl sulphate, stearic acid and mixtures thereof; preferably sodium stearyl fumarate.

The glidants selected from the group comprising colloidal silicon dioxide, colloidal anhydrous silica, talc, aluminium silicate or mixtures thereof; preferably colloidal silicon dioxide.

The sweeteners are selected from the group comprising aspartame, sucralose, saccharin, sugars such as glucose, lactose, fructose and sugar alcohols such as mannitol, sorbitol, xylitol, erythritol or mixtures thereof; preferably sucralose.

The flavouring agents are selected from the group comprising menthol, peppermint, cinnamon, chocolate, vanillin and fruit essences such as cherry, orange, strawberry, grape or mixtures thereof; preferably strawberry.

### Example 1: Wet granulation

| **ingredients** | **amount (%)** |
|---|---|
| Loxoprofen | 5 - 80% |
| Mannitol | 1 - 60 % |
| croscarmellose sodium | 0.1 - 50.0 % |
| Povidone K25 | 1 - 5 % |
| citric acid | 5.0 - 60.0 % |
| sodium bicarbonate | 5.0 - 60.0 % |
| sweetener | 0.01 - 3.00 % |
| flavouring agent | 0.5 - 5% |
| colloidal silicon dioxide | 0.01 - 5 % |
| sodyum stearyl fumarat | 0.25 - 3.0 % |

The production of the formulation is carried out as follows: loxoprofen, mannitol and 50% of croscarmellose sodium are taken into granulator and mixed. Granulation is achieved in povidone solution prepared with suitable amount of water. Granules are dried in fluid bed dryer or oven. Dried granules are sieved and citric acid, sodium bicarbonate, flavouring agent, sweetener, colloidal silicon dioxide and 50% of croscarmellose sodium are added. Mixed, then sodium stearyl fumarate was added and mixed again and then pressed into tablets.

### Example 2: Direct compression

| **Ingredients** | **% (amount)** |
|---|---|
| loxoprofen | 5 - 80 % |
| mannitol | 1 - 60 % |
| croscarmellose sodium | 0.1 - 50.0 % |
| citric acid | 5.0 - 60.0 % |
| sodium bicarbonate | 5.0 - 60.0 % |
| sweetener | 0.01 - 3.0 % |
| flavouring agent | 0.5 - 5.0 % |
| colloidal silicon dioxide | 0.01 - 5 % |
| sodium stearyl fumarate | 0.25 - 3.0 % |

The production of the formulation is carried out as follows: loxoprofen, mannitol, croscarmellose sodium, sodium bicarbonate and citric acid are mixed. Colloidal silicon dioxide, sweetener and flavouring agent is added to the powder mixture and mixed. Then, sodium stearyl fumarate, is added to the mixture and mixed again. Direct compression is performed with the final mixture.

## Claims

1. An orally disintegrating formulation comprising loxoprofen or a pharmaceutically acceptable salt thereof and croscarmellose sodium as a super disintegrant is present in an amount of 5 to 30 %, more preferably it is 5 - 15 % by weight of total formulation and one or more pharmaceutically acceptable excipients.

2. The orally disintegrating formulation according to claim 1, wherein the one or more pharmaceutically acceptable excipients are selected from the group comprising acidifying agents, alkalizing agents, diluents, taste masking coating, binders, lubricants, glidants, sweeteners and flavouring agents.

3. The orally disintegrating formulation according to claims 1, wherein the weight ratio of loxoprofen to croscarmellose sodium is in the range of 0.1 to 50 (w/w), preferably 2 to 30 (w/w) more preferably it is 2 to 20 (w/w).

4. The orally disintegrating formulation according to claim 1 to 4, the ratio of the acidifying agents to the alkalizing agents is between 20:1 (w/w) to 1:20 (w/w), preferably 10:1 (w/w) to 1:10 (w/w) and more preferably it is 5:1 (w/w) and 1:5 (w/w).

5. The orally disintegrating formulation according to claim 4, wherein the acidifying agent is selected from the group comprising citric acid, fumaric acid, adipic acid, acetic acid, hydrochloric acid, malic acid, nitric acid, phosphoric acid, sulfuric acid, tartaric acid and mixtures thereof, preferably it is citric acid.

6. The orally disintegrating formulation according to claim 4, wherein the alkalizing agent is selected from the group comprising sodium bicarbonate, sodium glycine carbonate, ammonia solution, ammonium carbonate, diethanolamine, diisopropanolamine, potassium hydroxide, sodium carbonate, sodium borate, sodium hydroxide, trolamine and mixtures thereof, preferably it is sodium bicarbonate.

7. The orally disintegrating formulation according to claim 4, wherein the diluents are selected from the group comprising mannitol, spray-dried mannitol, microcrystalline cellulose, lactose, starch, sodium carbonate, sodium bicarbonate, calcium carbonate and mixtures thereof; preferably the diluents is mannitol.

8. The orally disintegrating formulation according to claim 11, wherein the amount of mannitol is in the range of 1.0 to 60.0% by weight of total formulation; preferably it is 10.0 to 40.0% by weight of total formulation.
